# EUROPEAN PATENT APPLICATION

(11) **EP 1 429 558 A2**
(43) Date of publication of application: **16.06.2004**
(21) Application number: 03078731.1
(22) Date of filing: 28.11.2003
(51) Int. Cl.: H04N 7/16, H04N 7/18

(54) **Adaptive display system**

(30) Priority: 11.12.2002 US 316562
(71) Applicant: EASTMAN KODAK COMPANY, Rochester, New York 14650 (US)
(72) Inventor: Zacks, Carolyn A. c/o Eastman Kodak Company, Rochester New York 14650-2201 (US); Harel, Dan c/o Eastman Kodak Company, Rochester New York 14650-2201 (US); Marino, Frank c/o Eastman Kodak Company, Rochester New York 14650-2201 (US); Taxier, Karen M. c/o Eastman Kodak Company, Rochester New York 14650-2201 (US); Telek, Michael J. c/o Eastman Kodak Company, Rochester New York 14650-2201 (US)
(74) Representative: Haile, Helen Cynthia

(57) **Abstract**

A method for operating a display is provided. In accordance with the method, content is obtained and a profile is determined for the content. Elements are detected in a presentation space in which content presented by the display can be discerned. A profile is determined for each of the detected elements in the presentation space. Content is selected for presentation based upon the profiles for the detected elements and the content profile.

## Description

The present invention relates generally to display systems.

Large-scale video systems such as rear and front projection television systems, plasma displays, and other types of displays are becoming increasingly popular and affordable. Often such large scale video display systems are matched with surround sound and other advanced audio systems in order to present audio/visual content in a way that is more immediate and enjoyable for audience members. Many new homes and offices are even being built with media rooms or amphitheaters designed to accommodate such systems.

Increasingly, such large-scale video displays are also being usefully combined with personal computing systems and other information processing technologies such as internet appliances, digital cable programming, and interactive web based television systems that permit the displays to be used as part of advanced imaging applications such as videoconferencing, simulations, games, interactive programming, immersive programming and general purpose computing. In many of these applications, the large video displays are used to present information of a confidential nature such as financial transactions, medical records, and personal communications.

One inherent problem in the use of such large-scale display systems is that they present content on such a large visual scale that the content is observable over a very large presentation area. Accordingly, observers who may be located at a significant distance from the display system may be able to observe the content without the consent of the intended audience members. One way of preventing sensitive content from being observed by unintended audience members is to define physical limits around the display system so that the images presented on the display are visible only within a controlled area. Walls, doors, curtains, barriers, and other simple physical blocking systems can be usefully applied for this purpose. However, it is often inconvenient and occasionally impossible to establish such physical limits. Accordingly, other means are needed to provide the confidentiality and security that are necessary for such large scale video display systems to be used to present content that is of a confidential or sensitive nature.

Another approach is for the display to present images that are viewable within a very narrow range of viewing angles relative to the display. For example, a polarizing screen can be placed between the audience members and the display in order to block the propagation of image modulated light emitted by the display except within a very narrow angle of view. This approach is often not preferred because the narrow angle of view limits the range of positions at which people can observe the display.

Another approach involves the use of known displays and related display control programs that use kill buttons or kill switches that an intended audience member can trigger when an unintended audience member enters the presentation space A or the audience member feels that the unintended audience member is likely to enter the presentation space A. When the kill switch is manually triggered, the display system ceases to present sensitive content, and/or is directed to present different content. It will be appreciated that this approach requires that at least one audience member divide his or her attention between the content that is being presented and the task of monitoring the presentation space. This can lead to an unnecessary burden on the audience member controlling the kill switch.

Still another approach involves the use of face recognition algorithms. U.S. Pat. Appl. No. U.S. 2002/0135618 entitled "System And Method for Multi-Modal Focus Detection, Referential Ambiguity Resolution and Mood Classification Using Multi-Modal Input" filed by Maes et al. on Feb. 5, 2001 describes a system wherein face recognition algorithms and other algorithms are combined to help a computing system to interact with a user. In the approach described therein, multi-mode inputs are provided to help the system in interpreting commands. For example, a speech recognition system can interpret a command while a video system determines who issued the command. However, the system described therein does not consider the problem of preventing surreptitious observation of the contents of the display.

Thus what is needed is a display system and a display method for automatically adjusting the displayed content for privacy purposes.

In a first aspect of the present invention, what is provided is a method for operating a display. In accordance with the method, content is obtained and a profile is determined for the content. Elements are detected in a presentation space in which content presented by the display can be discerned. A profile is determined for each of the detected elements in the presentation space. Content is selected for presentation based upon the profiles for the detected elements and the content profile.

In another aspect of the present invention, a method for operating a display is provided. In accordance with the method, content for presentation is obtained and access privileges for the content are determined. At least one image of a presentation space within which the content is to be presented is obtained. Audience members are detected in the at least one image of the presentation space. Viewing privileges are determined for the detected audience members. The viewing privileges for the audience members are combined. Content for presentation is selected based upon the combined audience viewing privileges and the content viewing privileges.

In still another aspect of the present invention, a control system for a display is provided. The control system has a presentation space monitoring system generating a monitoring signal indicative of conditions in a presentation space within which content presented by the display can be discerned and a processor adapted to receive the content and to determine a profile for the content. The processor is further adapted to detect elements in the presentation space based upon the monitoring signal and to determine a profile for each element in the presentation space. The processor selects content for presentation based upon profiles for the detected elements and the content profile.

In a further aspect of the present invention, a control system for a display adapted to present content to a presentation space is provided. The control system has a presentation space imaging means for capturing an image of the presentation space and an element profile means for detecting elements in the presentation space image and determining a profile for each detected element and a content profile means for determining a profile for the content. A processor is adapted to select content for presentation based upon the element profiles and the content profile.

Fig. 1 shows a block diagram of one embodiment of an adaptive display system of the present invention.

Fig. 2 shows a flow diagram of one embodiment of a method for presenting images in accordance with present invention.

Fig. 3 shows a block diagram of another embodiment of an adaptive display system of the present invention.

Fig. 4 is an illustration of the use of one embodiment of the present invention for video conferencing.

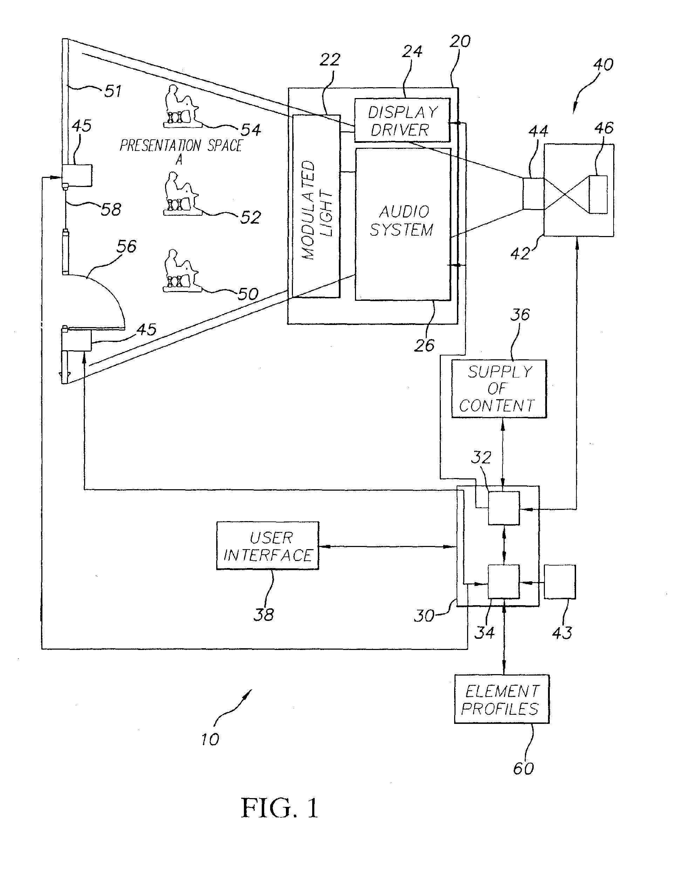

Fig. 1 shows a first embodiment of a presentation system 10 of the present invention that adaptively presents content. As used herein, the term content refers to any form of video, audio, text, affective or graphic information or representations and any combination thereof.

In the embodiment shown in Fig. 1, presentation system 10 comprises a display device 20 such as an analog television, a digital television, computer monitor, projection system or other apparatus capable of receiving signals containing images or other visual content and converting the signals into an image that can be discerned in a presentation space A. Display device 20 comprises a source of image modulated light 22 such as a cathode ray tube, a liquid crystal display, an organic light emitting display, an organic electroluminescent display, or other type of display element. Alternatively, the source of image modulated light 22 can comprise any front or rear projection display system, holographic and/or immersive type display systems known in the art. A display driver 24 is also provided. Display driver 24 receives image signals and converts these image signals into control signals that cause the source of image modulated light 22 to display an image.

Presentation system 10 also comprises an audio system 26. Audio system 26 can comprise a conventional monaural or stereo sound system capable of presenting audio components of the content in a manner that can be detected throughout presentation space A. Alternatively, audio system 26 can comprise a surround sound system which provides a systematic method for providing more than two channels of associated audio content into presentation space A. Audio system 26 can also comprise other forms of audio systems that can be used to direct audio to specific portions of presentation space A. One example of such a directed audio system is described in commonly assigned U.S. Patent Application Serial No. 09/467,235, entitled "Pictorial Display Device With Directional Audio" filed by Agostinelli et al. on December 20, 1999.

Presentation system 10 also incorporates a control system 30. Control system 30 comprises a signal processor 32 and a controller 34. A supply of content 36 provides a content bearing signal to signal processor 32. Supply of content 36 can comprise for example a digital videodisc player, videocassette player, a computer, a digital or analog video or still camera, scanner, cable television network, the Internet or other telecommunications system, an electronic memory or other electronic system capable of conveying a signal containing content for presentation. Signal processor 32 receives this content and adapts the content for presentation. In this regard, signal processor 32 extracts video content from a signal bearing the content and generates signals that cause the source of image modulated light 22 to display the video content. Similarly, signal processor 32 extracts audio signals from the content bearing signal. The extracted audio signals are provided to audio system 26 which converts the audio signals into an audible form that can be heard in presentation space A.

Controller 34 selectively causes images received by signal processor 32 to be presented by the source of image modulated light 22. In the embodiment shown in Fig. 1, a user interface 38 is provided to permit local control over various features of the display device 20. For example, user interface 38 can be adapted to allow one or more audience members to enter system adjustment preferences such as hue, contrast, brightness, audio volume, content channel selections etc. Controller 34 receives signals from user interface 38 that characterize the adjustments requested by the user and will provide appropriate instructions to signal processor 32 to cause images presented by display device 20 to take on the requested system adjustments.

Similarly, user interface 38 can be adapted to allow a user of presentation system 10 to enter inputs that enable or disable presentation system 10 and/or to select particular channels of content for presentation by the system 10. User interface 38 can provide other inputs for use in calibration as will be described in greater detail below. For example, user interface 38 can be adapted with voice recognition module that recognizes audible output and provides recognition into signals that can be used by controller 34 to control operation of the device.

A presentation space monitoring system 40 is also provided to sample presentation space A to detect elements in presentation space A that can influence whether certain content should be presented. As is noted above, presentation space A will comprise any space or area in which the content presented by the presentation system 10 can be discerned. Presentation space A can take many forms. For example, in the embodiment shown in Fig. 1, content presented by display device 20 is limited by wall 51. Alternatively, where presentation system 10 is operated in an open space such as a display area in a retail store, a train station or an airport terminal presentation space A will be limited by the optical display capabilities of presentation system 10. Similarly where, presentation system 10 is operated in a mobile environment, presentation space A can change as presentation system 10 is moved.

In the embodiment shown in Fig. 1, presentation space monitoring system 40 comprises a conventional image capture device such as an analog or digital image capture unit 42 comprising a taking lens unit 44 that focuses light from a scene onto an image sensor 46 that converts the light into electronic signal. Taking lens unit 44 and image sensor 46 cooperate to capture images that include presentation space A.

Images captured by image capture unit 42 are supplied to signal processor 32. Signal processor 32 analyzes the images to detect image elements in the images that are captured of presentation space A. Examples of image elements that can be found in presentation space A include audience member 50, 52, and 54 or things such as door 56 or window 58 or other items (not shown) that may have an influence on what is presented by presentation system 10. Such other items can include content capture devices such as video cameras, digital still cameras, or any other image capture device as well as audio capture devices.

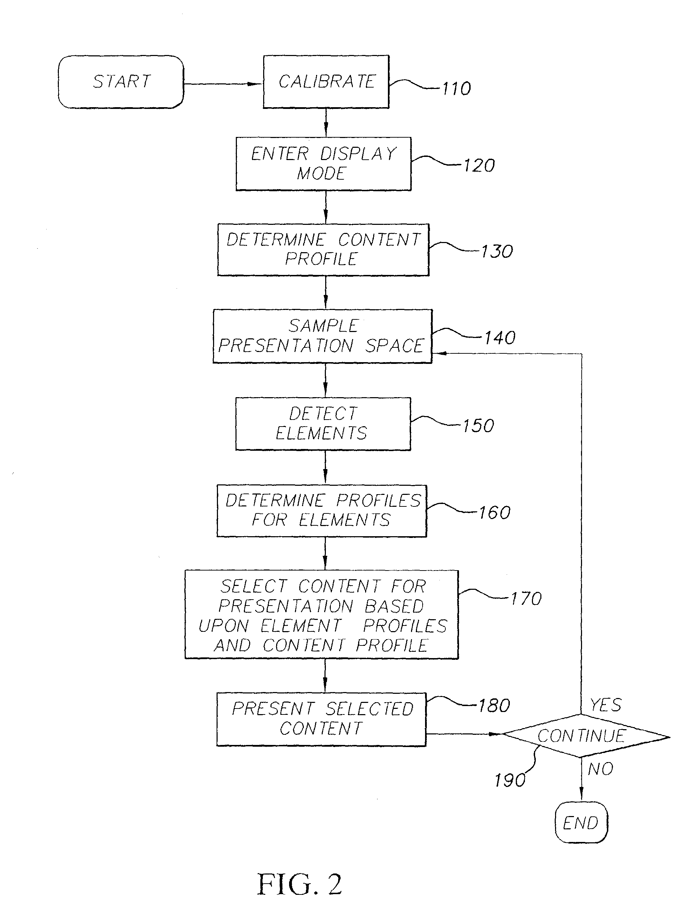

A source of element profiles 60 is provided. The source of element profiles 60 can be a memory device such as an optical, magnetic or electronic storage device or a storage device provided by the remote network. The source of element profiles 60 can also comprise an algorithm for execution by a processor such as image processor 32 or controller 34. Such an algorithm determines profile information based upon analysis of the elements found in the presentation space image captured by image capture unit 42 and assigns a profile to the identified elements as will now be described with reference to Fig. 2.

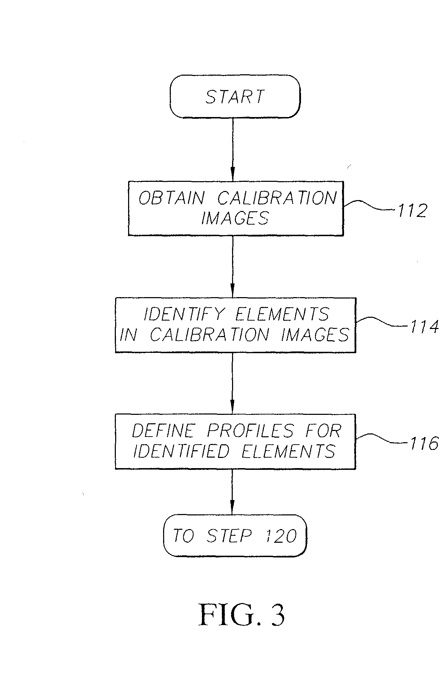

Fig. 2 shows a flow diagram of one embodiment of a method for operating a presentation system such as presentation system 10. As is shown in Fig. 2, the presentation system is initially calibrated (step 110). As is shown in Fig. 3, during calibration, calibration images including images of presentation space A are obtained (step 112). A user of presentation system 10 uses the calibration images to identify elements that are or that can be present in presentation space A (step 114) and a profile is defined for each element (step 116).

The elements identified during calibration can include, for example, people such as audience member 50, 52 and 54 who are present in presentation space A. Such people can be identified using face recognition or other software to analyze the image or images of presentation space. In this regard, the calibration images used during calibration can include images of particular people or their specific characteristics which can be used by the face recognition software to help identify the people who are likely to be in the presentation space. Profile information is assigned to each person. The profile identifies the nature of the content that the person is entitled to observe. For example, where it is determined that the person is an adult audience member, the viewing privileges may be broader than the viewing privileges associated with a child audience member. In another example, an audience member may have access to selected information relating to the adult that is not available to other adult audience members. The profile can assign viewing privileges in a variety of ways. For example, viewing privileges can be defined with reference to ratings such as those provided by the Motion Picture Association of America (MPAA), Encino, C.A., U.S.A. which rates motion pictures and assigns general ratings to each motion picture. Where this is done each element is associated with one or more ratings and the viewing privileges associated with the element are defined by the ratings with which it is associated. However, it will also be appreciated that it is possible to assign profiles without individually identifying audience member 50, 52 and 54. This is done by classifying people and assigning a common set of privileges to each class. Where this is done, profiles can be assigned to each class of viewer. For example, people in presentation space A can be classified as adults and children with one set of privileges associated with the adult class of audience members and another set of privileges associated the child class.

Elements other than people can also be assigned profile information. Items such as windows, doors, blinds, curtains and other objects in a presentation space A can be assigned with a profile. For example, door 56 can be assigned with a profile that describes one level of display privileges when the image indicates that the door is open, another set when the door is partially open and still another set of privileges when the door is closed.

In another example, window 58 can be assigned with a profile that provides various viewing privileges associated with the condition of the window. For example the window and profile that defines one set of privileges when no observer is detected outside of window 58 and another set of privileges when an observer is detected outside of the window 58.

In still another example, the portions of the presentation space A imaged by presentation space monitoring system 40 that do not frequently change such as carpet areas, furniture etc., can also be identified as static area elements. Static area elements can be assigned with profiles that identify viewing privileges that are enabled when the static area elements change during presentation of the image.

In a further example, various portions of presentation space A imaged by image capture unit 42 that are expected to change during display of the content but wherein the changes are not frequently considered to be relevant to a determination of the privileges associated with the content can be identified. For example, a large grandfather clock (not shown) could be present in the scene. The clock has turning hands on its face and a moving pendulum. Accordingly where content is presented over a period of time, changes will occur in the appearance of the clock. However, these changes are not relevant to a determination of the viewing privileges. Thus, these areas are identified as dynamic elements and a profile is assigned to each dynamic element that indicates that changes in the dynamic element are to be ignored in determining what content to present.

Finally, it may be useful to define a set of privilege conditions for presentation space A when unknown elements are present in presentation space A. For example, it may be useful to define a profile for an "unknown" element. The unknown element profile is used to define privilege settings where an unknown person or undefined change in an element occurs.

It will be appreciated that, although the calibration process has been described as a manual calibration process, the calibration process can also be performed in an automatic mode by scanning a presentation space to search for predefined classes of elements and for predefined classes of users.

Once calibrated, presentation system 10 determines a desire to view content and enters a display mode (step 120). Typically this desire is indicated using user interface 38. However, presentation system 10 can be automatically activated with controller 34 determining that presentation system 10 should be activated because, for example, controller 34 is programmed to activate presentation system 10 at particular times of the day, or because, for example, controller 34 determines that a new signal has been received for presentation on the display.

Signal processor 32 analyzes signals bearing content and determines access privileges associated with this content (step 130). The access privileges identify a condition or set of conditions that are recommended or required to view the content. For example, MPAA ratings can be used to determine access privileges. Alternatively, the access privileges can be determined by analysis of the proposed content. For example, where the display is called upon to present digital information such as from a computer, the content of the information can be analyzed based upon the information contained in the content and a rating can be assigned. Access privileges for a particular content can also be manually assigned during calibration.

In still another alternative, an audience member can define certain classes of content that the audience member desires to define access privileges for. For example, the audience member can define higher levels of access privileges for private content. When the content is analyzed, scenes containing private content can be identified by analysis of the content or by analysis of the metadata associated with the content that indicates the content has private aspects. Such content can then be automatically associated with appropriate access privileges. The presentation space A is then sampled (step 140). In this embodiment, this sampling is performed when image capture unit 42 captures an image of presentation space A. Depending on the optical characteristics of presentation space monitoring system 40, it may be necessary to capture different images at different depths of field so that the images obtained depict the entire presentation space with sufficient focus to permit identification of elements in the scene.

The image or images are then analyzed to detect elements in the image (step 150). Image analysis can be performed using pattern recognition or other known image analysis algorithms. Profiles for each element in the image are then obtained based on this analysis (step 160).

The content that is to be presented to presentation space A is then selected (step 170). Where more than one element is identified in presentation space A, this step involves combining the element profiles. There are various ways in which this can be done. The element profiles can be combined in an additive manner with each of the element profiles examined and content selected based upon the sum of the privileges associated with the elements. Table I shows an example of this type. In this example three elements are detected in the presentation space, an adult, a child and an open door. Each of these elements has an assigned profile identifying viewing privileges for the content. In this example, the viewing privileges are based upon the MPAA ratings scale.

| Viewing Privilege Type (Based On MPAA Ratings) | Element I: Adult Profile | Element II: Child Profile | Element III: Open Door Profile | Combined Privileges |
|---|---|---|---|---|
| G - General Audiences | YES | YES | YES | YES |
| PG - Parental Guidance Suggested | YES | YES | NO | YES |
| PG-13 - Parents Strongly Cautioned | YES | NO | NO | YES |

As can be seen in this example, the combined viewing privileges include all of the viewing privileges of the adult even though the child element and the open door element have fewer viewing privileges.

The profiles can also be combined in a subtractive manner. Where this is done profiles for each element in the presentation space are examined and the privileges for the audience are reduced for example, to the lowest level of privileges associated with one of the profiles for one of the elements in the room. An example of this is shown in Table II. In this example, the presentation space includes the same adult element, child element and open door element described with reference to Fig. 1.

| Viewing Privilege Type (Based On MPAA Ratings) | Element I: Adult Profile | Element II: Child Profile | Element III: Open Door Profile | Combined Privileges |
|---|---|---|---|---|
| G - General Audiences | YES | YES | YES | YES |
| PG - Parental Guidance Suggested | YES | YES | NO | NO |
| PG-13 - Parents Strongly Cautioned | YES | NO | NO | NO |

However, when the viewing privileges are combined in a subtractive manner, the combined viewing privileges are limited to the privileges of the element having the lowest set of privileges: the open door element. Other arrangements can also be established. For example, profiles can be determined by analysis of content type such as violent content, mature content, financial content or personal content with each element having a viewing profile associated with each type of content. As a result of such combinations, a set of element viewing privileges is defined which can then be used to make selection decisions.

Content is then selected for presentation based upon the combined profile for the elements and the profile for the content (step 170). The combined element profiles yield a set of viewing privileges. This set of viewing privileges can be compared to privilege information derived from the content profile. Content having a set of access privileges that correspond to the set of viewing privileges is selected for presentation. In the example shown in Table I, content having a PG rating can be selected for presentation because the PG rating corresponds to the combined viewing privileges which include G, PG, and PG-13 rated content. Conversely, in the example shown in Table II, the same content having a PG rating cannot be presented because the PG rating does not correspond to the combined viewing privileges, which, in the case of Table II, are limited to a G rating. As noted above the viewing privileges and access privileges can be assigned in different ways. Accordingly the selection process can be performed in different ways.

For example, where content is received in streams such as multiple cable channels, selected programming, or selected channels can be blocked. Where the content comprises a single stream of content such as a movie that is recorded on a digital videodisk, selected videodisks and/or selected portions of the content can be excised. Financial and other text-based information can be identified by text based context analysis and blocked in whole, or particularly sensitive portions can be excised.

In one alternative embodiment, a primary stream of content is available having portions that are associated with a reduced set of access privileges and portions that are associated with a greater set of access privileges. A secondary stream of content is available having portions of content that correspond to the portions of the primary stream having the greater set of access privileges but with content modified to have a lower set of access privileges. In this embodiment, the step of selecting content for presentation comprises determining that set of the viewing privileges do not correspond to the greater set of access privileges associated with the portions of the primary stream of content and selecting for presentation content from the secondary stream of content to substitute for such portions of the primary stream.

The selected content is then presented (step 180) and the process repeats until it is desired to discontinue the presentation of the content (step 190). During each repetition, changes in composition of the elements presentation space can be detected. Such changes can occur, for example, as people move about in the presentation space. Further, when such changes are detected the way in which the content is presented can be automatically adjusted to accommodate this change. For example, when an audience member moves from one side of the presentation space to another side of the presentation space, then presented content such as text, graphic, and video elements in the display can change relationships within the display to optimize the viewing experience.

Other user preference information can be incorporated into the element profile. For example, as is noted above, presentation system 10 is capable of receiving system adjustments by way of user interface 38. In one embodiment, these adjustments can be entered during the calibration process (step 110) and presentation space monitoring system 40 can be adapted to determine which audience member has entered what adjustments and to incorporate the adjustment preferences with the profile for an image element related to that audience member. During operation, an element in presentation space A is determined to be associated with a particular audience member, signal processor 32 can use the system adjustment preferences to adjust the presented content. Where more than one audience member is identified in presentation space A, the system adjustment preferences can be combined and used to drive operation of presentation system 10.

As described above, presentation space monitoring system 40 comprises a single image capture unit 42. However, presentation space monitoring system 40 can also comprise more than one image capture unit 42.

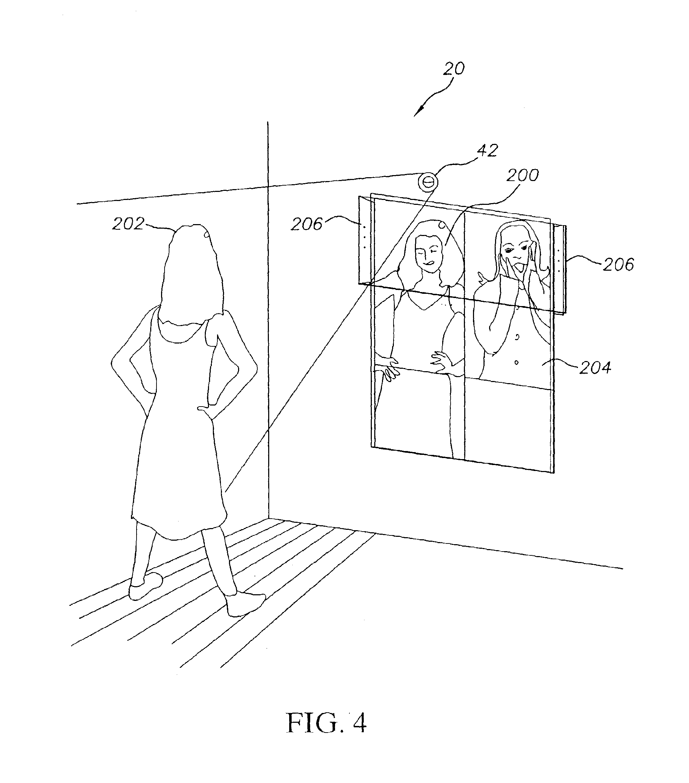

As is shown in Fig. 4, presentation system 10 can be usefully applied for the purpose of video-conferencing. In this regard, audio system 26, user interface 38 and image capture unit 42 can be used to send and receive audio, video and other signals that can be transmitted to a compatible remote video conferencing system. In this application, presentation system 10 can receive signals containing content from the remote system and present video portions of this content on display device 20. As is shown in this embodiment, display device 20 provides a reflective image portion 200 showing user 202 a real reflected image or a virtual reflected image derived from images captured of presentation space A. A received content portion 204 of display device 20 shows video portions of the received content. The reflective image portion 200 and received content portion 204 can be differently sized or dynamically adjusted by user 202. Audio portions of the content are received and presented by audio system 26, which, in this embodiment includes speaker system 206.

In the above-described embodiments, the presentation space monitoring system 40 has been described as sampling presentation space A using image capture unit 42. However, presentation space A can be sampled in other ways. For example, presentation space monitoring system 40 can use other sampling systems such as a conventional radio frequency sampling system 43. In one popular form, elements in the presentation space are associated with unique radio frequency transponders. Radio frequency sampling system 43 comprises a transceiver that emits a polling signal to which transponders in the presentation space respond with self-identifying signals. The radio frequency sampling system 43 identifies elements in presentation space A by detecting the signals. Further, radio frequency signals in the presentation space such as those typically emitted by recording devices can also be detected. Other conventional sensor systems 45 can also be used to detect elements in the presentation space and/or to detect the condition of elements in the presentation space. Such detectors include switches and other transducers that can be used to determine whether a door is open or closed or window blinds are open or closed. Elements that are detected using such systems can be assigned with a profile during calibration in the manner described above with the profile being used to determine combined viewing privileges. Image capture unit 42, radio frequency sampling system 43 and sensor systems 45 can also be used in combination in a presentation space monitoring system 40.

In certain installations, it may be beneficial to monitor areas outside of presentation space A but proximate to presentation space A to detect elements such as people who may be approaching the presentation space. This permits the content on the display or audio content associated with the display to be adjusted before the presentation space A is encroached or entered such as before audio content can be detected. The use of multiple image capture unit 42 may be usefully applied to this purpose as can the use of a radio frequency sampling system 43 or a sensor system 45 adapted to monitor such areas.

It will be appreciated that the present invention, while particularly useful for improving the confidentiality of information presented by a large scale video display system, is also useful for other smaller systems such as video displays of the types used in video cameras, personal digital assistants, personal computers, portable televisions and the like.

The invention has been described in detail with particular reference to certain preferred embodiments thereof, but it will be understood that variations and modifications can be effected within the spirit and scope of the invention. In this regard it will be appreciated that the various components of the presentation system 10 shown in Fig. 1 can be combined, separated and/or combined with other components to provide the claimed features and functions of the present invention.

## Claims

1. A method for operating a display, the method comprising the steps of:
obtaining content;
determining a profile for the content;
detecting elements in a presentation space within which content presented by the display can be discerned;
determining a profile for each of the detected elements in the presentation space; and
selecting content for presentation based upon the profiles for the detected elements and the content profile.

2. The method of claim 1, wherein the step of detecting elements in the presentation space comprises capturing an image of the presentation space and analyzing the image to detect the elements.

3. The method of claim 1, wherein the step of detecting elements in the presentation space comprises detecting radio frequency signals from transponders in the presentation space and identifying elements in the presentation space based upon the detected radio frequency signals.

4. The method of claim 1, wherein the step of detecting elements in the presentation space comprises detecting signal from sensors adapted to monitor the presentation space and identifying elements in the presentation space based upon the detected signals.

5. The method of claim 1, wherein each element profile contains viewing privileges and the content profile contains access privileges wherein the step of selecting content for presentation based upon the profiles comprises combining the viewing privileges in an additive manner and selecting content for presentation based upon the combined viewing privileges and the access privileges.

6. The method of claim 1, wherein the content comprises alternative streams of content with access privileges associated with each stream of content and wherein the step of selecting content for presentation comprises the steps of determining viewing privileges based upon the element profiles and incorporating content from the alternative streams of content for presentation as a single output stream, wherein the content having access privileges corresponding to the viewing privileges is incorporated in the single output stream.

7. A method for operating a display, the method comprising the steps of:
obtaining content for presentation;
determining access privileges for the content;
obtaining at least one image of a presentation space within which the content is to be presented;
detecting the audience members in the at least one image of the presentation space;
determining audience member viewing privileges for the detected audience members;
combining the viewing privileges for the audience members;
selecting content for presentation based upon the combined audience viewing privileges and the content viewing privileges; and
presenting the selected content.

8. A control system for a display, the control system comprising:
a presentation space monitoring system generating a monitoring signal representative of conditions in a presentation space within which content presented by the display can be discerned; and
a processor adapted to receive the content and to determine a profile for the content, with the processor further adapted to detect elements in the presentation space based upon the monitoring signal and to determine a profile for each element in the presentation space;
wherein the processor selects content for presentation based upon the profiles for the detected elements and the content profile.

9. The control system of claim 8, wherein the presentation space monitoring system further monitors areas outside of the presentation space and the processor detects elements that are positioned to enter the presentation space and wherein the processor selects content for presentation based upon the profiles for the detected elements in the presentation space and the detected elements that are positioned to encroach upon the presentation space.

10. A control system for a display adapted to present content to a presentation space, the control system comprising:
a presentation space imaging means for capturing an image of the presentation space;
an element profile means for detecting elements in the presentation space image and determining a profile for each detected element;
a content profile means for determining a profile for the content; and
a processor adapted to select content for presentation by the display based upon the element profile and the content profile.
